# FASCICULE DE BREVET EUROPEEN

(11) **EP 2 067 009 B1**
(45) Date de publication et mention de la délivrance du brevet: **15.01.2014**
(21) Numéro de dépôt: 07848230.4
(22) Date de dépôt: 13.09.2007
(51) Int. Cl.: G01G 19/44, G01G 23/00, A61B 5/103

(54) **APPAREIL POSTURAL DYNAMIQUE PERMETTANT UNE DÉTECTION D'UNE POSTURE BIPÈDE ÉQUILIBRÉE**
DYNAMISCHE POSITIONIERUNGSVORRICHTUNG ZUR ERKENNUNG DER POSITION EINES BALANCIERTEN ZWEIBEINERS
DYNAMIC POSITIONAL APPARATUS FOR DETECTING A BALANCED BIPED POSITION

(30) Priorité: 22.09.2006 FR 0608323
(43) Date de publication de la demande: 10.06.2009
(73) Titulaire: Walthert, Nicole, 45000 Orleans (FR)
(72) Inventeur: Walthert, Nicole, 45000 Orleans (FR)
(74) Mandataire: Hirsch & Associés
(86) Numéro de dépôt international: PCT/FR2007/001490
(87) Numéro de publication internationale: WO 2008/034965

(56) Documents cités:
- EP-A- 0 519 836
- WO-A-87/01923
- FR-A- 2 619 702
- FR-A- 2 639 111
- FR-A1- 2 648 559
- FR-A1- 2 725 022
- FR-A2- 2 647 331
- US-A- 3 906 931
- US-A- 4 598 717
- US-A- 4 726 435
- US-B2- 6 563 059

## Description

La présente invention se rapporte au domaine des appareils personnels indicateurs d'informations de poids et/ou de répartition de poids, en particulier le domaine de la pesée d'une personne en posture verticale dynamique. L'invention concerne plus particulièrement, par utilisation du principe de répartition du poids du corps sur les appuis plantaires en position d'équilibre bipède, un appareil et un procédé permettant d'afficher le poids du corps en équilibre sur une surface plane et horizontale (quelle que soit la posture verticale : debout, semi accroupie ou accroupie).

On sait que, dans de telles conditions, le poids du corps se répartit sur tous les points d'appui des deux voûtes plantaires. Chaque pied possède trois points d'appui, deux à l'avant (têtes du premier et cinquième métatarsien) et un à l'arrière (tubérosités postérieures du calcanéum). En position d'équilibre bipède sur un sol plan et horizontal, le poids du corps s'applique par moitié sur chaque clé de voûte plantaire, centre du cou-de-pied, situé à l'aplomb du prolongement du bord antérieur de la jambe, avant de se répartir ensuite par moitié sur les points d'appui antérieurs et le point d'appui postérieur de la voûte plantaire. Dans une telle position verticale debout, le centre de gravité du corps humain (troisième vertèbre lombaire) et la ligne de gravité sont correctement placés, la base de sustentation (contour des pieds et espace entre les pieds) déterminée, les membres inférieurs sont dans le prolongement du tronc et les courbures de la colonne vertébrale atteignent leur forme caractéristique. Dans ces circonstances d'équilibre bipède, le poids du corps se répartit en quatre quarts sur les points d'appui plantaires. Chaque avant et arrière pied reçoit le quart du poids total du corps.

De façon connue, l'architecture du corps humain, squelette, tendrons, parties molles permet un équilibre debout confortable et statique sur les points d'appui plantaires. Dans ces conditions, l'activité musculaire du tronc et des membres inférieurs est faible. L'équilibre obtenu est passif, transitoire et instable.

On sait que, dès qu'il y a perte d'équilibre, soit risque de chute et quelle qu'en soit la direction, des contractions musculaires réflexes compensatrices, tonus de posture, rétablissent l'équilibre. Une fois l'équilibre atteint, l'activité des muscles anti-gravitaires cesse alors, jusqu'à la prochaine perte d'équilibre. L'activité musculaire du tronc et des membres inférieurs est faible. L'équilibre obtenu est passif, transitoire et instable.

Par ailleurs, il est bien établi que dans toutes les positions d'accroupissement vertical bipède, membres inférieurs fléchis, articulations déverrouillées, l'équilibre est maintenu grâce à l'action puissante des muscles anti-gravitaires des membres inférieurs et de la colonne. Dans ces conditions, le centre de gravité s'abaisse, l'équilibre est plus stable, la ligne de gravité passe par le centre de chaque cou-de-pied, la voûte plantaire s'affaisse, la longueur des pieds augmente. Les points d'appui glissent légèrement, ceux de l'avant vers l'avant du pied, ceux de l'arrière vers l'arrière du pied et la répartition du poids du corps sur les points d'appui est la même qu'en position verticale debout. Plus les positions accroupies sont basses, plus l'équilibre est stable et plus le tonus des muscles anti-gravitaires est élevé. Dans la position d'accroupissement complet, le centre de gravité est au plus bas, l'équilibre est très stable, le corps, replié sur lui-même prend une forme foetale, genoux en avant, fesses en arrière, tête en avant, colonne courbée vers l'avant sur toute sa longueur (les différentes lignes de courbure de colonne sont effacées), et l'activité des muscles anti-gravitaires cesse. La répartition du poids du corps sur les points d'appui plantaire reste inchangée. Cette position est une posture de détente du corps adoptée par les enfants et de nombreux adultes dans certains pays.

C'est le système nerveux central qui assure et régule le tonus musculaire de posture, à partir des informations sensorielles proprioceptives (muscles, tendons, articulations), vestibulaires (labyrinthes) et extéroceptives visuelles, auditives et tactiles, qu'il reçoit. Parvenir à l'état d'équilibre exige la mise en oeuvre d'un système complexe à plusieurs entrées (stimulations sensorielles) et plusieurs sorties (réactions musculaires). Toute modification d'une stimulation (entrée) entraîne une réaction de rééquilibrage dynamique (sortie). Le résultat est une posture harmonieuse et efficace.

On sait que tout mouvement part de l'équilibre et y aboutit. Il est également connu que les trois sensibilités extéroceptives (vue, ouïe, toucher plantaire) en rapport avec le monde extérieur, agissent sur le tonus de posture en réglant nos attitudes et que la sensibilité des récepteurs tactiles plantaires joue ici, un rôle majeur. Soumis à la pression du poids du corps et à ses variations d'une part, en contact avec le sol, sa texture, ses aspérités, ses dénivellations, d'autre part, les récepteurs des corpuscules tactiles plantaires, par le nombre d'informations qu'ils détectent et transmettent au système nerveux, sont les starters des réflexes assurant l'équilibre.

Le toucher plantaire est un organe sensoriel dont l'acuité est entretenue essentiellement par la marche et les accroupissements. La marche, locomotion naturelle symétrique, offre l'avantage, pendant la phase de déroulement du pied sur le sol, de stimuler, à tour de rôle, la perception tactile de chaque point d'appui plantaire. Les accroupissements bipèdes activent fortement la perception de tous les appuis plantaires pendant les variations d'écrasement des voûtes plantaires. Depuis une cinquantaine d'années, notre mode de vie a changé, ce qui a entraîné une réduction du temps de marche quotidien en dessous d'un seuil indispensable à l'entretien de la perception de la sensibilité tactile de nos appuis plantaires et des réflexes de posture verticale qui en découlent. L'homme des sociétés modernes, de plus en plus sédentaire (assis), perd peu à peu, sans s'en rendre compte, la perception de ses appuis plantaires en même temps qu'il perd son tonus de posture verticale et l'habitude de marcher de façon naturelle.

L'usure anormale et fréquente des semelles de chaussure (par exemple uniquement au niveau du talon) signe des défauts d'appui pendant la déambulation : peu ou pas de pression sur certains appuis et trop de pression sur d'autres. En conséquence, les réflexes d'équilibre sont perturbés. Certains de ces réflexes sont moins bien activés et perdent en efficacité dynamique. Le tonus de certains muscles anti-gravitaires (membres inférieurs et colonne) diminue. Par perte de tonus dans les membres inférieurs (cuisses principalement), l'accroupissement devient pénible. Nombreux sont ceux qui y renoncent. Ainsi disparaissent progressivement, par défaut d'entretien de la sensibilité des appuis plantaires (marche et accroupissement notamment) la perception de cette sensibilité, le dynamisme postural, la coordination des gestes (tout geste part d'un ou plusieurs points d'appui plantaires), souplesse et harmonie de la forme du corps en même temps qu'apparaissent des symptômes d'obésité. Face à ce constat, il est recommandé par tous les médecins de surveiller son poids et de bouger, marcher ou pratiquer une activité sportive.

Un objet de l'invention est, en réactivant la perception de la sensibilité tactile de tous ses appuis plantaires, starters dynamiques de la posture verticale, de permettre à l'homme sédentarisé de, non seulement bouger, marcher, s'accroupir ou pratiquer un sport en toute sécurité, mais aussi de lui en redonner l'envie et de lui éviter ainsi des risques d'obésité.

Un objet de l'invention est de permettre à un utilisateur de se concentrer sur la répartition du poids de son corps sur ses appuis plantaires en situation de verticalité (en appui sur une surface plane, mobile), en lui faisant prendre conscience de son déséquilibre.

Il est connu dans l'art antérieur des systèmes à deux plateaux mobiles permettant une visualisation de la répartition du poids de l'utilisateur sur chacune de ses jambes.

Le document WO 87/01923, du même inventeur, divulgue un appareil à plateau unique mobile monté sur des ressorts et doté d'un indicateur d'horizontalité de ce plateau mobile. Cet appareil permet à l'utilisateur de corriger activement les appuis de ses deux pieds pour atteindre l'équilibre, visualisable par l'indicateur d'horizontalité du plateau. Un niveau à bulle permet d'indiquer précisément l'atteinte de l'équilibre.

Il est possible d'insérer un pèse-personne dans un évidement de l'appareil décrit dans le document WO 87/01923. L'ensemble résultant permet alors d'indiquer de façon constante le poids tandis que l'utilisateur peut continuer à améliorer ses appuis sur le plateau mobile. Un inconvénient de cet appareil connu est que l'indication de l'équilibre n'est pas interactive et suscite insuffisamment l'envie de l'utilisateur d'adopter de bonnes postures pour lesquelles l'équilibre est atteint.

Le document FR 2 647 331, du même inventeur, enseigne le même appareil que celui du document WO 87/01923 auquel des capteurs de déplacement ont été ajoutés entre chaque ressort à mi-distance. Ce système n'est qu'une amélioration du niveau à bulle qui est remplacé par des diodes électroluminescentes et ne permet pas d'améliorer la détermination de la position du corps disposé sur l'appareil en s'affranchissant de la compression des ressorts et de la charge du corps en appui sur le plateau mobile.

Il est connu, dans un tout autre domaine, d'utiliser des dalles à résistance dépendant de la force dites FSR (Force Sensitive Resistance), dotées d'une ou deux sortie(s) analogique(s) à 3 points pour être branchées à un module de conversion-traitement qui sert à interpréter des mouvements du corps humain. Les dalles à deux sorties analogiques sont sensibles à la posture de l'individu sur la dalle, et plus précisément à la répartition de son poids dans l'espace. Elles sont totalement indépendantes du poids lui-même, ce qui leur permet de réagir exactement de la même façon avec un enfant ou avec une personne de 90 kilogrammes. Ces dalles sont par exemple utilisées pour former un sol sensitif dans le but par exemple de moduler les sons et les lumières.

Cependant, ces dalles FSR ne sont ni utilisées ni utilisables pour établir des diagnostics d'équilibre.

Le document FR 2 619 702 divulgue un plateau rendu mobile, par rapport à un système placé sur le sol, à l'aide une unique liaison rotule. L'information ou réponse fournie par l'appareil est statique et est surtout perçue de façon visuelle. Seul un mouvement global de rotation est généré par la liaison rotule pour s'opposer à l'équilibre du patient, ce qui limite fortement la stimulation de la perception tactile des appuis plantaires.

Le document US 4,598,717 enseigne un appareil pour déterminer les défauts posturaux et de quantifier ces défauts. Cet appareil permet seulement de quantifier des défauts mais ne permet pas d'améliorer la détermination de la position du corps disposé sur l'appareil.

Le document US 6,563,059 décrit une balance permettant de mesurer le taux de graisse et de mesurer le poids d'un utilisateur. Cette balance ne permet que de mesurer mais ne permet pas d'améliorer la détermination de la position du corps disposé sur l'appareil.

La présente invention a donc pour objet de pallier un ou plusieurs des inconvénients de l'art antérieur en définissant un appareil doté de fonctions indicatrices supplémentaires, activables selon que l'équilibre est atteint ou non, l'appareil restant simple de conception et permettant de rendre compte précisément de l'équilibre dans la répartition du poids sur les appuis plantaires.

A cet effet, l'invention concerne un appareil selon l'une des revendications 1 à 15 et 17.

Ainsi, il est permis selon l'invention d'obtenir un affichage d'une information telle que le poids par utilisation de capteurs plus ou moins sollicités en fonction de la répartition des forces exercées sur le plateau par l'appui des pieds, ce qui permet avantageusement de détecter via le circuit de traitement l'atteinte ou non de l'équilibre par la personne. Cet équilibre est atteint de façon dynamique en raison de la présence des organes déformables tels que des ressorts qui sont sans cesse sollicités. Le sujet doit faire preuve de concentration et peut, par pression sur ses appuis, corriger son déséquilibre, de sorte que sa perception de tous les appuis plantaires est réactivée de façon aisée, ludique et efficace.

Un autre objet de l'invention est de fournir un procédé pour ajouter, dans un pèse-personne conventionnel, une fonction de détection d'anomalies d'équilibre du corps humain.

A cet effet, l'invention concerne un procédé d'intégration, dans un pèse-personne à plateau et indication visuelle et/ou sonore du poids, d'une fonction de détection d'anomalies d'équilibre du corps humain, selon la revendication 16.

L'invention, avec ses caractéristiques et avantages, ressortira plus clairement à la lecture de la description faite en référence aux dessins annexés donnés à titre d'exemples non limitatifs dans lesquels :
- la figure 1 représente une vue en perspective et en transparence d'une forme de réalisation d'un pèse-personne conforme à l'invention,
- la figure 2 représente une vue de dessus d'une forme de réalisation d'un pèse-personne conforme à l'invention,
- les figures 3A et 3B montrent des exemples de réalisation qui diffèrent par le plateau-support,
- la figure 4 illustre un exemple de capteur de force utilisé dans le pèse-personne selon l'invention,
- la figure 5 montre un exemple de positionnement des pieds sur le plateau mobile de l'appareil,
- la figure 6 illustre un exemple d'affichage lors d'une utilisation de l'appareil.

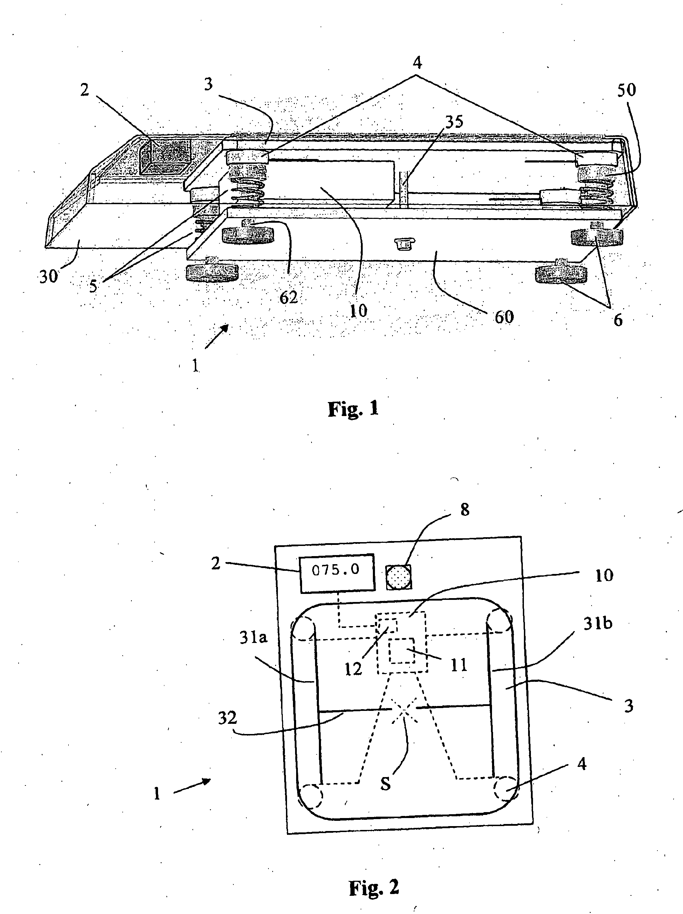

L'appareil peut former un pèse-personne (1) comme illustré dans les figures 1 et 2. Il comprend un boîtier (30) muni de moyens d'appui au sol (6) tels que des pieds, un plateau (3) mobile relié au boîtier (30), des capteurs (4) de force prévus entre lesdits moyens d'appui (6) et le plateau (3) pour fournir chacun un signal électrique représentatif d'une force détectée par le capteur (4). Ce signal peut être une tension. Les moyens d'appui au sol comprennent par exemple quatre pieds disposés sous le boîtier (30), les pieds étant rendus solidaires d'un socle fixe (60). Le plateau (3), rigide, est positionné sous la partie supérieure du boîtier (30) et repose sur le socle fixe (60) par l'intermédiaire d'organes déformables (5) tels que des ressorts ou éléments élastiques équivalents. Les ressorts sont choisis pour supporter des poids élevés. Ainsi, un ensemble de quatre ressorts peut supporter une charge très supérieure à 100 kilogrammes par exemple. Le plateau (3) sert de support pour les pieds de l'utilisateur. Dans l'exemple de la figure 1, le boîtier (30) comprend une fenêtre supérieure pour laisser apparaître l'affichage du poids (2) et le niveau (8).

Dans un mode de réalisation préféré de l'invention, les capteurs (4) sont identiques et répartis dans des zones symétriques entre elles par rapport à un axe de symétrie central (S), comme illustré notamment à la figure 2. Cet axe central (S) du plateau (3) est vertical lorsque le pèse-personne est placé sur un sol horizontal. Des moyens de réglage (8, 62) de l'horizontalité du boîtier (30) sont prévus en cas de défaut d'horizontalité des sols, de façon à rendre l'axe de symétrie central (S) vertical. Un ou plusieurs pieds peuvent être munis d'un système (62) à molette ou système équivalent (à rotule,..) pour ajuster l'horizontalité du boîtier (30). Le niveau à bulle (8), solidaire du boîtier (30), permet d'indiquer l'horizontalité du boîtier (30). Dans une variante, le niveau à bulle (8) peut être remplacé par un pendule associé à des capteurs détectant les déplacements du pendule pour permettre d'indiquer l'horizontalité.

Le pèse-personne (1) est doté d'au moins une unité de traitement (10) pour déterminer à partir des signaux des capteurs (4) le poids total d'un utilisateur du pèse-personne placé sur le plateau (3). Ce poids total peut par exemple se déduire de la somme des signaux de sortie des capteurs (4), après une conversion analogique-numérique. Des moyens d'affichage (2) de type connus en soi sont prévus pour indiquer le poids total déterminé. Les organes déformables (5) sont associés à chacune des zones à capteurs (4) pour soutenir selon une direction ascendante le plateau (3) et lesdits capteurs (4) reliés au boîtier (30). Une mauvaise répartition des forces selon les zones peut avantageusement être détectée par un circuit de traitement formant un module (11) de détection de déséquilibre. Ce module (11) utilise les données des capteurs (4) reçues par l'unité de traitement (10) pour détecter, par comparaison, une mauvaise répartition des forces reçues sur les différentes zones à capteurs (4).

Les organes déformables (5) exercent chacun une force de réaction correspondant exactement à la part de poids reçue. Ils permettent ainsi à l'utilisateur, dès qu'il se place sur le plateau (3), de ressentir les différences de réaction au niveau de ses appuis plantaires, sans qu'il n'y ait de stimulations autres que celles provoquées par son propre poids. Contrairement à un appareil de pesée conventionnel qui ne donne le poids de l'utilisateur qu'en situation passive, l'appareil selon l'invention permet de donner le poids en verticalité dynamique. Cet appareil permet en effet de réveiller les sensations d'appui de l'utilisateur, de mettre en jeu les réflexes de verticalité efficaces et de développer le tonus de tous les muscles anti-gravitaires (membres inférieurs et tronc).

Lorsqu'une mauvaise répartition des forces est détectée par le module (11), ce dernier fournit l'information à l'interface (12) de contrôle des moyens d'affichage (2). Dans ce cas, l'affichage du poids est muet. A titre d'exemple, lorsque l'interface (12) de contrôle reçoit une information d'équilibre issue du module (11) de détection, l'interface autorise aussitôt un affichage du poids total de l'utilisateur du pèse-personne. Lorsque des différences entre les forces mesurées sont détectées par le module (11) de détection de déséquilibre, alors l'affichage peut rester muet ou afficher une information par défaut autre que le poids mesuré. Le module de détection (11) et l'interface (12) de contrôle peuvent être implémentés dans les moyens de traitement de l'unité de traitement (10).

Dans un mode de réalisation de l'invention, le boîtier (30) est muni à ses quatre coins, au droit des pieds, de quatre éléments servant de guides aux organes déformables (5) formés par les ressorts. Les quatre organes déformables (5) à ressort sont donc chacun alignés verticalement avec un des pieds. Les ressorts avantageusement en acier ou autre matériau de résistance comparable sont de hauteur et de tare égale et d'une résistance suffisante à l'écrasement pour éviter que le plateau (3) ne puisse venir en butée contre le socle (60) quand il supporte sa charge. Le plateau (3) est de dimension analogue à celle du socle (60) dans l'exemple de la figure 1 et repose par ses quatre coins sur des protubérances (50) à épaulement renversé. La partie supérieure des ressorts s'appuie sur l'épaulement renversé de ces protubérances (50).

Les capteurs (4) de force sont répartis sous quatre coins du plateau (3). Dans l'exemple de la figure 1, un organe vertical (35) relié au socle fixe (60) est disposé dans le boîtier (30) pour éviter le débattement vertical du plateau (3). Cet organe vertical (35) peut consister en une tige traversant le plateau par un trou pratiqué dans ce dernier. Le trou est central et suffisamment grand par rapport aux dimensions de la section de la tige pour permettre au plateau (3), mobile, d'avoir un degré de liberté suffisant pendant toutes ses variations d'inclinaison. Au-delà d'un seuil d'environ 5°, le plateau vient buter sur l'organe vertical au niveau du trou central.

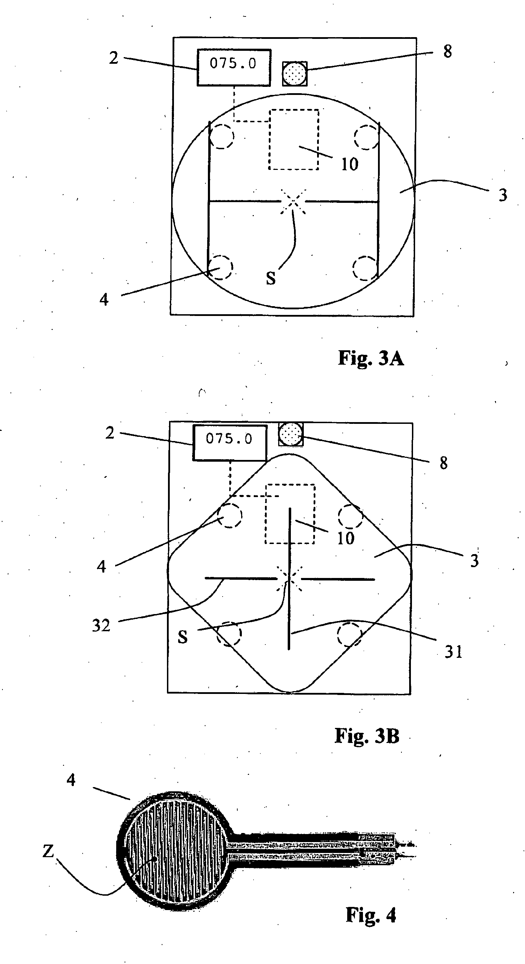

En référence à la figure 1, les capteurs (4) peuvent être insérés entre les protubérances (50) ou éléments de butée analogues et le plateau (3). Les capteurs (4) sont par exemple des résistances de détection de force FSR (Force Sensitive Resistance). Leur structure est extrêmement simple, comme illustré à la figure 4 : il s'agit de deux feuilles de polymère(s) laminées ensemble (leur épaisseur ne dépasse pas 0,75 mm). Chaque capteur (4) de type FSR répond à une force en faisant décroître sa résistance électrique (ohmique) d'autant plus que l'on appuie plus fort sur le composant transducteur (Z). L'une des feuilles de polymère est recouverte d'un réseau d'électrodes et l'autre feuille est recouverte d'un matériau semiconducteur. Peu sensible au bruit et aux vibrations, sa large plage d'impédance permet l'emploi d'une électronique d'interface simplifiée. De façon connue en soi, l'interface mécanique des capteurs (4) de force utilisés prévoit un support suffisamment "ferme" et résistant. A titre d'exemple non limitatif, des capteurs de force FSR commercialisés par la société Interlink Electronics® peuvent être utilisés pour traduire en signaux électriques les forces reçues dans les différentes parties du plateau (3). D'autres types de capteurs de force équivalents peuvent naturellement être utilisés.

En référence aux figures 2, 3A et 3B, on comprend que la répartition des forces s'effectue en quatre zones réparties autour de l'axe de symétrie (S) du plateau (3) et cela, quelle que soit la forme envisagée pour le plateau (3). Le plateau (3) peut tout aussi bien être globalement rectangulaire ou carré comme dans les figures 2 et 3B, ou ovoïde/circulaire comme dans la figure 3A. Les capteurs doivent simplement être bien répartis dans chacune de ces quatre zones associées respectivement aux zones des appuis plantaires.

Dans un mode de réalisation des figures 2, 3A et 3B, le plateau (3) porte des éléments de marquage pour l'emplacement des deux pieds. Par exemple, les éléments de marquage comprennent au moins une ligne médiane transversale (32) par rapport à deux des capteurs (4) de force. Cette ligne médiane (32) indique à l'utilisateur qu'il doit placer chacun de ses pieds à cheval sur cette ligne médiane, de façon à ce que chaque cou-de-pied (partie du pied correspondant à la clé de voûte plantaire) soit à l'aplomb de cette ligne médiane (32). D'autres lignes (31a, 31b) sagittales sont matérialisées sur le plateau (3). Elles indiquent l'emplacement du bord externe de chaque pied (cinquième métatarsien) et permettent de placer chaque pied dans la position qu'il convient d'adopter. Dans la variante de la figure 3B, le plateau (3) comporte une ligne axiale (31) séparant la zone dédiée au pied droit de la zone dédiée au pied gauche. Orthogonalement à cette ligne axiale (31) peut être tracée une ligne médiane (32) pour séparer la zone dédiée aux avant-pieds de la zone dédiée aux arrière-pieds.

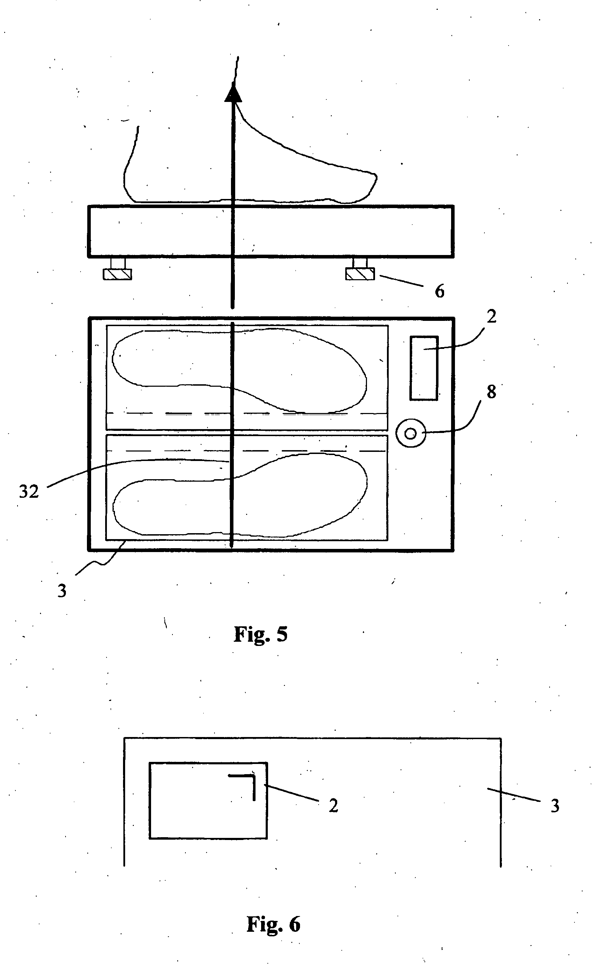

L'appareil peut donner, par rapport à une grille de positionnement sur le plateau (3), une indication de l'origine du déséquilibre : déséquilibre vers l'avant, vers l'arrière, vers un côté, à la fois sur un côté et vers l'avant ou vers l'arrière. En référence à la figure 6, cette indication est donnée par des traits lumineux faisant partie d'une interface d'affichage de caractères numériques ou alphanumériques. Le poids peut être affiché par cette interface uniquement en cas d'équilibre. L'affichage combiné d'un trait du haut et d'un trait de droite est réalisé lorsque la répartition des forces mesurées est déséquilibrée à la fois vers l'avant et vers la droite. L'afficheur (2) est placé sur la partie avant de l'appareil, par exemple au niveau du plateau (3) ou peut être surélevé et branché de façon filaire ou non au circuit de traitement de l'appareil.

Comme illustré à la figure 5, l'utilisateur doit se mettre dans une position déterminée (après réglage de l'horizontalité du plateau (3)) par rapport au plan d'appui, en suivant les lignes ou grille de positionnement. Les pieds sont placés de façon symétrique plateau et le devant de la jambe est à l'aplomb de la ligne médiane (32) transversale. Dans un mode de réalisation de l'invention, les moyens d'affichage (2) affichent un message de démarrage pour inciter l'utilisateur à rechercher la position d'équilibre. Ensuite, l'afficheur peut inciter l'utilisateur à effectuer un mouvement : accroupissement, coup(s) de poing, remontée à partir de la position accroupie, balancement des bras, etc.

Alternativement ou de surcroît, un affichage du poids est réalisé à chaque fois que la position d'équilibre est atteinte. Dans une forme de réalisation, une programmation peut être réalisée pour indiquer un gain au bénéfice de l'utilisateur (qui gagne un cadeau par exemple ou l'affichage d'une séquence ludique), dans le cas où il serait parvenu à maintenir la position d'équilibre pendant une durée déterminée, par exemple 30 ou 60 seconde. Tenir l'équilibre longtemps n'est pas évident car quatre éléments élastiques distincts sont simultanément sollicités. L'appareil permet de vérifier que l'utilisateur a son centre de gravité aligné avec à l'axe de symétrie central du plateau (3). Dans la position débout, un tel alignement se traduit par l'obtention d'une posture véritablement droite et verticale. L'appareil proposé selon l'invention est utilisé pour la correction et le contrôle de la verticalité dans différentes positions verticales bipèdes, debout, semi accroupie ou accroupie. Tous les avantages obtenus avec l'appareil décrit dans le brevet EP 0 238 586-B1 du même inventeur sont obtenus avec le pèse-personne selon l'invention. Il est naturellement possible d'utiliser les fonctions de comparaison du module de détection (11) pour permettre une visualisation sur les moyens d'affichage des variations de l'équilibre. L'affichage peut par exemple représenter une bulle d'un niveau à bulle virtuel, le déplacement de la bulle étant lié à la répartition des forces détectées. Les signaux délivrés par les capteurs sont alors utilisés pour modifier la position d'une image représentative d'une bulle qui se déplace autour d'une cible correspondant à l'atteinte de l'équilibre. L'atteinte de l'équilibre peut être une condition préalable au calcul ou à l'affichage du poids. Des moyens connus en soi pour déterminer le pourcentage de graisse peuvent être associés à l'unité de traitement (10) et ce pourcentage peut aussi être affiché, par exemple à la suite de l'indication du poids.

Le pèse-personne peut aussi comporter une interface de communication à courte distance pour transmettre des données à un dispositif d'affichage librement mobile par rapport au reste du pèse-personne. Cette interface de type radiofréquence, Bluetooth®, Wifi, IR (infrarouge) ou autre assure la communication avec le dispositif indépendant d'affichage, doté d'un récepteur adapté.

Dans une forme de réalisation alternative ou complémentaire, des notes peuvent être émises par une sortie audio reliée à l'unité de traitement (10). Par exemple une note comme "la" peut correspondre à l'atteinte de l'équilibre, tandis que des notes plus basses ou plus hautes peuvent être diffusées en cas de déséquilibre vers un côté ou un autre.

Dans un mode de réalisation de l'invention, le module (11) de détection de déséquilibre dispose d'un seuil de détection prédéterminé. Ainsi, lorsque la différence entre la plus petite et la plus grande des forces mesurées reste inférieure au seuil, par exemple de 1 kilogramme, le module de détection ne va pas détecter un déséquilibre. On comprend que le module (11) de détection est capable d'effectuer une comparaison entre des valeurs représentatives des forces mesurées pour chaque zone à capteur(s) de force (4). Une opération de détection peut être réalisée en comparant les valeurs obtenues deux à deux, en déterminant ainsi la différence maximale, puis en comparant cette différence maximale au seuil de détection. Une interface utilisateur (non représentée) peut être prévue pour permettre de paramétrer ce seuil. Le dépassement du seuil de détection signifie que l'équilibre du corps humain n'est pas obtenu et les moyens d'affichage (2) permettent d'en informer l'utilisateur. Un indicateur sonore peut aussi être ajouté pour émettre des sons ou informations auditives qui dépendent de la répartition des forces sur le plateau, donc du bon équilibre ou non du corps humain. Le mode d'indication sonore peut même se substituer au mode d'indication par affichage sur un écran. Des moyens autonomes d'alimentation électrique, tels que des accumulateurs d'un type connu, sont prévus à l'intérieur du boîtier (30) comme source d'alimentation des moyens d'affichage et/ou de l'indicateur sonore.

Le pèse-personne (1) peut être doté d'une interface utilisateur incluant des moyens de sélection entre notamment les deux modes de fonctionnement suivants :
- un mode conventionnel d'affichage du poids total de l'utilisateur du pèse-personne, quelle que soit la répartition des forces détectées par chacun des capteurs (4) ; et
- un mode dynamique d'affichage du poids total de l'utilisateur du pèse-personne, dans lequel l'interface (12) de contrôle interdit l'affichage dudit poids total tant qu'une mauvaise répartition des forces est détectée par le module (11) de détection.

Ainsi, l'utilisateur peut personnaliser via une interface de l'appareil, le mode de fonctionnement du pèse-personne (1).

Un des avantages de l'appareil selon l'invention est qu'il fait appel à la concentration de l'utilisateur qui doit trouver sa verticalité, quelle que soit la position verticale qu'il choisit, debout, semi accroupie ou accroupie, s'il veut connaître son poids et/ou faire cesser les indications relatives au déséquilibre. Pouvant être guidé par les déplacements de la bulle d'un niveau solidaire du plateau, l'utilisateur découvre sur quelles parties du pied il doit exercer une pression pour recentrer la bulle et voir l'affichage de son poids A l'usage, l'appareil forme un système réellement dynamique qui permet de mettre en interaction et en opposition, lors de la recherche de l'équilibre, les forces de réaction des organes déformables (par exemples des ressorts) avec les diverses forces d'appui du sujet placé sur le plateau (3).

Lorsque l'appareil forme un pèse-personne (1), la pesée affichée est le résultat d'un petit exercice facile, dynamique, ludique, incitatif et aucunement dangereux, exercice pendant lequel la sensibilité tactile de tous les appuis plantaires est activée. Après sa pesée dynamique, l'utilisateur est surpris de se sentir plus droit, plus léger et d'aplomb sur ses pieds et plus à l'aise pendant ses déplacements. Il ressent une sensation oubliée perçue dans l'enfance, le confort de la verticalité et n'a qu'une envie : bouger.

L'invention peut avantageusement permettre d'intégrer, dans un pèse-personne (1) à plateau (3) et indication visuelle et/ou sonore du poids, une fonction de détection d'anomalies d'équilibre du corps humain. Pour cela il suffit par exemple :
- de prévoir des capteurs de force (4) répartis de façon symétrique par rapport à un axe de symétrie central (S) du plateau du pèse-personne (1) ;
- d'associer, à chacun des capteurs (4) de force, un organe déformable (5) pour soutenir selon une direction ascendante une partie du plateau (3) et le capteur (4) associé ;
- mettre en liaison les capteurs (4) avec un module (11) de comparaison qui permet de détecter la répartition des forces ; et
- prévoir une interface (12) de contrôle des moyens d'indication de poids pour générer une indication visuelle et/ou sonore modifiée lorsqu'une mauvaise répartition des forces est détectée par le module (11) de détection, la modification permettant ainsi d'indiquer à un utilisateur des anomalies d'équilibre liées à de mauvais appuis.

L'utilisateur d'un appareil conforme à l'invention ressent un appui correct, confortable lorsqu'il visualise son poids. Cet aplomb au sol est ainsi particulièrement bien perçu par l'utilisateur qui est alors incité à s'exercer au maximum (concentration, mise en jeu de ses réflexes, en ne se contentant pas d'un équilibre statique, passif) pour trouver rapidement sa verticalité. Il doit être évident pour les personnes versées dans l'art que la présente invention permet des modes de réalisation sous de nombreuses autres formes spécifiques sans l'éloigner du domaine d'application de l'invention comme revendiqué. En particulier, les capteurs de force peuvent être réalisés sous la forme d'une dalle FSR qui donne la répartition de poids selon deux axes. On comprend qu'il suffit par exemple de bien répartir les capteurs suivant quatre zones du plateau (3) mobile, chacune des quatre zones étant dédiée à la détection d'un appui avant ou arrière de l'un des pieds.

## Revendications

1. Appareil (1) pour détecter et corriger des anomalies d'équilibre du corps humain, comportant un plateau (3) support, sur lequel peut prendre place un utilisateur, et au moins une pièce de référentiel (60) positionnable sur une surface sensiblement plane, le plateau (3) étant superposé par rapport à la pièce de référentiel (60), ledit plateau (3) étant soutenu par au moins trois organes déformables (5) élastiques agissant chacun suivant une direction orthogonale par rapport à un plan défini par ladite pièce de référentiel (60), les organes déformables étant répartis de façon symétrique par rapport à un axe de symétrie central du plateau (3), l'appareil comprenant :
- une unité de traitement (10) dotée d'un circuit de traitement ;
**caractérisé en ce que** l'appareil comprend en outre :
- un capteur de force (4) placé à une des extrémités de chacun des organes déformables (5), chaque capteur (4) délivrant un signal envoyé au circuit de traitement, le circuit de traitement détectant un équilibre entre les signaux représentant les forces et correspondant à une position d'équilibre parfaite par rapport au centre de symétrie du plateau (3) ou détectant un déséquilibre entre ces signaux ; et
- des moyens pour générer l'affichage d'une information en cas d'équilibre et une indication visuelle et/ou sonore modifiée lorsqu'une mauvaise répartition des forces est détectée par le circuit de traitement, l'indication visuelle et/ou sonore indiquant à l'utilisateur des anomalies d'équilibre et incitant l'utilisateur à rechercher la position d'équilibre.

2. Appareil selon la revendication 1, **caractérisé en ce que** l'information affichée en cas d'équilibre est le poids de l'utilisateur, l'appareil définissant ainsi un pèse-personne.

3. Appareil selon la revendication 2, comprenant :
- un module (11) de détection de déséquilibre incluant ledit circuit de traitement pour détecter par comparaison, à partir de données des capteurs (4) reçues par l'unité de traitement (10), une mauvaise répartition des forces détectées par chaque capteur (4) ; et
- une interface (12) de contrôle des moyens d'affichage (2) permettant de modifier l'affichage lorsqu'une mauvaise répartition des forces est détectée par le module (11) de détection.

4. Appareil selon la revendication 2 ou 3, comportant un boîtier (30) muni de moyens d'appui au sol (6), le plateau (3) étant relié au boîtier (3), des capteurs (4) de force prévus entre lesdits moyens d'appui (6) et le plateau (3) pour fournir chacun un signal électrique représentatif d'une force détectée par le capteur (4), au moins une unité de traitement (10) pour déterminer à partir des signaux des capteurs (4) le poids total d'un utilisateur du pèse-personne placé sur le plateau (3), et des moyens d'affichage (2) permettant d'indiquer le poids total déterminé, les capteurs (4) étant identiques et répartis dans des zones symétriques entre elles par rapport à un axe de symétrie central (S), les organes déformables (5) étant associés à chacune des zones de capteurs (4) pour soutenir selon une direction ascendante le plateau (3) et lesdits capteurs (4) relativement au boîtier (30).

5. Appareil selon la revendication 4, comprenant des moyens de réglage (8, 62) de l'horizontalité du boîtier (30).

6. Appareil selon les revendications 4 ou 5, dans lequel les moyens (6) d'appui au sol comprennent quatre pieds disposés sous le boîtier (30), les pieds étant solidaires d'un socle fixe formant l'élément référentiel (60).

7. Appareil selon la revendication 3 seule ou combinée à l'une des revendications 4 à 6, dans lequel ladite interface (12) de contrôle autorise un affichage du poids total de l'utilisateur du pèse-personne en fonction de la délivrance par le module (11) de détection d'une information représentative d'une détection d'équilibre.

8. Appareil selon la revendication 7, comprenant quatre organes déformables (5) élastiques chacun alignés verticalement avec un des pieds.

9. Appareil selon la revendication 7 ou 8, dans lequel les capteurs (4) de force sont répartis sous quatre coins du plateau (3) et au moins un organe vertical (35) relié à l'élément référentiel (60) est disposé dans le boîtier (30) pour limiter le débattement du plateau (3) supporté à l'aide des organes déformables (5).

10. Appareil selon la revendication 3 seule ou combinée à l'une des revendications 4 à 9, dans lequel le module (11) de détection de déséquilibre dispose d'un seuil de détection prédéterminé tel qu'une différence entre la plus petite et la plus grande des forces mesurées qui reste inférieure au seuil ne permet pas de détecter un déséquilibre.

11. Appareil selon la revendication 3 seule ou combinée à l'une des revendications 4 à 10, comprenant des moyens de sélection entre deux modes de fonctionnement :
- un mode conventionnel d'affichage du poids total de l'utilisateur du pèse-personne, quelle que soit la répartition des forces détectées par chacun des capteurs (4) ; et
- un mode dynamique d'affichage du poids total de l'utilisateur du pèse-personne, dans lequel ladite interface (12) de contrôle interdit l'affichage dudit poids total tant qu'une mauvaise répartition des forces est détectée par le module (11) de détection.

12. Appareil selon une des revendications 1 à 11, dans lequel les capteurs (4) de force sont des capteurs à résistance électrique variable.

13. Appareil selon la revendication 4 seule ou combinée à l'une des revendications 5 à 12, dans lequel un niveau à bulle (8) est solidaire du boîtier (30) pour indiquer l'horizontalité du boîtier (30).

14. Appareil selon une des revendications 1 à 13, dans lequel le plateau (3) porte des éléments de marquage pour l'emplacement des deux pieds, les éléments de marquage comprenant au moins une ligne médiane par rapport à deux des capteurs (4) de force.

15. Appareil selon une des revendications 1 à 13, comprenant une interface de communication à courte distance pour transmettre des données à un dispositif d'affichage librement mobile par rapport au reste de l'appareil.

16. Procédé d'intégration, dans un pèse-personne (1) à plateau (3) et indication visuelle et/ou sonore du poids, d'une fonction de détection d'anomalies d'équilibre du corps humain, **caractérisé en ce qu'**il comprend :
- une étape de répartition de capteurs de force (4) de façon symétrique par rapport à un axe de symétrie central (S) du plateau du pèse-personne (1) ;
- une étape d'association, à chacun des capteurs (4) de force, d'un organe déformable (5) pour soutenir selon une direction ascendante une partie du plateau (3) et le capteur (4) associé ;
- une étape de connexion aux capteurs (4) d'un module (11) de comparaison pour détecter la répartition des forces ; et
- une étape d'intégration d'une interface (12) de contrôle des moyens d'indication de poids pour générer une indication visuelle et/ou sonore modifiée lorsqu'une mauvaise répartition des forces est détectée par le module (11) de détection, la modification d'indiquant à l'utilisateur des anomalies d'équilibre et incitant l'utilisateur à rechercher la position d'équilibre.

17. Appareil selon une des revendications 1 à 15 comprenant des moyens pour générer l'affichage incitant l'utilisateur à effectuer un mouvement.

## Patentansprüche

1. Gerät (1) zur Erfassung und Korrektur von Gleichgewichtsstörungen des menschlichen Körpers, umfassend eine Stützplatte (3), auf der ein Benutzer Platz nehmen kann, und mindestens einen Referenzteil (60), der auf einer im Wesentlichen ebenen Fläche positionierbar ist, wobei die Platte (3) in Bezug zu dem Referenzteil (60) darüber liegend angeordnet ist, wobei die Platte (3) durch mindestens drei verformbare elastische Elemente (5) gestützt wird, die jeweils in eine Richtung orthogonal zu einer von dem Referenzteil (60) definierten Ebene wirken, wobei die verformbaren Elemente symmetrisch in Bezug zu einer zentralen Symmetrieachse der Platte (3) verteilt sind, wobei das Gerät umfasst:
- eine Bearbeitungseinheit (10), die mit einer Bearbeitungsschaltung versehen ist;
**dadurch gekennzeichnet, dass** das Gerät ferner umfasst:
- einen Kraftsensor (4), der an einem der Enden jedes der verformbaren Elemente (5) angeordnet ist, wobei jeder Sensor (4) ein Signal liefert, das an die Bearbeitungsschaltung gesandt wird, wobei die Bearbeitungsschaltung ein Gleichgewicht zwischen den Signalen, die die Kräfte repräsentieren und einer perfekten Gleichgewichtsposition in Bezug zum Symmetriemittelpunkt der Platte (3) entsprechen, oder ein Ungleichgewicht zwischen diesen Signalen erfasst; und
- Mittel, um die Anzeige einer Information im Falle eines Gleichgewichts und eine veränderte visuelle und/oder akustische Anzeige zu erzeugen, wenn eine schlechte Verteilung der Kräfte von der Bearbeitungsschaltung erfasst wird, wobei die visuelle und/oder akustische Anzeige dem Benutzer Gleichgewichtsstörungen anzeigt und den Benutzer dazu veranlasst, die Gleichgewichtsposition zu suchen.

2. Gerät nach Anspruch 1, **dadurch gekennzeichnet, dass** die angezeigte Information im Falle eines Gleichgewichts das Gewicht des Benutzers ist, wobei das Gerät somit eine Personenwaage definiert.

3. Gerät nach Anspruch 2, umfassend:
- ein Modul (11) zur Erfassung eines Ungleichgewichts, einschließlich der Bearbeitungsschaltung, um durch Vergleich auf Basis von Daten der Sensoren (4), die von der Bearbeitungseinheit (10) erhalten werden, eine schlechte Verteilung der Kräfte, die von jedem Sensor (4) erfasst werden, zu erfassen; und
- eine Kontrollschnittstelle (12) der Anzeigemittel (2), die es ermöglicht, die Anzeige zu verändern, wenn eine schlechte Verteilung der Kräfte von dem Erfassungsmodul (11) erfasst wird.

4. Gerät nach Anspruch 2 oder 3, umfassend ein Gehäuse (30), das mit Mitteln zur Abstützung am Boden (6) versehen ist, wobei die Platte (3) mit dem Gehäuse (30) verbunden ist, Kraftsensoren (4), die zwischen den Abstützungsmitteln (6) und der Platte (3) vorgesehen sind, um jeweils ein elektrisches Signal zu liefern, das für eine vom Sensor (4) erfasste Kraft repräsentativ ist, mindestens eine Bearbeitungseinheit (10), um auf Basis der Signale der Sensoren (4) das Gesamtgewicht eines Benutzers der auf der Platte (3) angeordneten Personenwaage zu bestimmen, und Anzeigemittel (2), die es ermöglichen, das bestimmte Gesamtgewicht anzugeben, wobei die Sensoren (4) identisch und in zueinander symmetrischen Zonen in Bezug zu einer zentralen Symmetrieebene (S) verteilt sind, wobei die verformbaren Elemente (5) jeder der Zonen von Sensoren (4) zugeordnet sind, um entlang einer aufsteigenden Richtung die Platte (3) und die Sensoren (4) in Bezug zum Gehäuse (30) zu unterstützen.

5. Gerät nach Anspruch 4, umfassend Mittel zur Einstellung (8, 62) der horizontalen Ausrichtung des Gehäuses (30).

6. Gerät nach den Ansprüchen 4 oder 5, bei dem die Mittel (6) zur Abstützung am Boden vier Füße umfassen, die unter dem Gehäuse (30) angeordnet sind, wobei die Füße mit einem festen Sockel verbunden sind, der das Referenzelement (60) bildet.

7. Gerät nach Anspruch 3 alleine oder in Kombination mit einem der Ansprüche 4 bis 6, bei dem die Kontrollschnittstelle (12) eine Anzeige des Gesamtgewichts des Benutzers der Personenwaage in Abhängigkeit von der Übermittlung einer für eine Gleichgewichtserfassung repräsentativen Information durch das Erfassungsmodul (11) gestattet.

8. Gerät nach Anspruch 7, umfassend vier verformbare elastische Elemente (5), die jeweils vertikal mit einem der Füße ausgerichtet sind.

9. Gerät nach Anspruch 7 oder 8, bei dem die Kraftsensoren (4) unter vier Ecken der Platte (3) verteilt sind, und mindestens ein mit dem Referenzelement (60) verbundenes vertikales Element (35) in dem Gehäuse (30) angeordnet ist, um den Ausschlag der Platte (3), die mit Hilfe der verformbaren Elemente (5) getragen wird, zu begrenzen.

10. Gerät nach Anspruch 3 alleine oder in Kombination mit einem der Ansprüche 4 bis 9, bei dem das Modul (11) zur Erfassung eines Ungleichgewichts über eine vorbestimmte Erfassungsschwelle verfügt, so dass es eine Differenz zwischen der kleinsten und der größten der gemessenen Kräfte, die kleiner als die Schwelle bleibt, nicht ermöglicht, ein Ungleichgewicht zu erfassen.

11. Gerät nach Anspruch 3 alleine oder in Kombination mit einem der Ansprüche 4 bis 10, umfassend Mittel zur Auswahl zwischen zwei Funktionsmodi:
- einem konventionellen Anzeigemodus des Gesamtgewichts des Benutzers der Personenwaage, unabhängig von der Verteilung der von jedem der Sensoren (4) erfassten Kräfte; und
- einen dynamischen Anzeigemodus des Gesamtgewichts des Benutzers der Personenwaage, bei dem die Kontrollschnittstelle (12) die Anzeige des Gesamtgewichts untersagt, solange eine schlechte Verteilung der Kräfte vom Erfassungsmodul (11) erfasst wird.

12. Gerät nach einem der Ansprüche 1 bis 11, bei dem die Kraftsensoren (4) Sensoren mit variablem elektrischem Widerstand sind.

13. Gerät nach Anspruch 4 alleine oder in Kombination mit einem der Ansprüche 5 bis 12, bei dem eine Wasserwaage (8) mit dem Gehäuse (30) verbunden ist, um die horizontale Ausrichtung des Gehäuses (30) anzuzeigen.

14. Gerät nach einem der Ansprüche 1 bis 13, bei dem die Platte (3) Markierungselemente für die Stelle der beiden Füße trägt, wobei die Markierungselemente mindestens eine Mittellinie in Bezug zu zwei der Kraftsensoren (4) umfassen.

15. Gerät nach einem der Ansprüche 1 bis 13, umfassend eine Kurzstreckenkommunikationsschnittstelle, um Daten an eine Anzeigevorrichtung zu übertragen, die in Bezug zum übrigen Gerät frei beweglich ist.

16. Verfahren, um in eine Personenwaage (1) mit Platte (3) und visueller und/oder akustischer Anzeige des Gewichts eine Funktion zur Erfassung von Gleichgewichtsstörungen des menschlichen Körpers zu integrieren, **dadurch gekennzeichnet, dass** es umfasst:
- einen Schritt der Verteilung von Kraftsensoren (4) symmetrisch zu einer zentralen Symmetrieachse (S) der Platte der Personenwaage (1);
- einen Schritt der Verbindung eines verformbaren Elements (5) mit jedem der Kraftsensoren (4), um entlang einer aufsteigenden Richtung einen Teil der Platte (3) und den zugehörigen Sensor (4) zu unterstützen;
- einen Schritt der Verbindung eines Vergleichsmoduls (11) mit den Sensoren (4), um die Kraftverteilung zu erfassen; und
- einen Schritt der Integration einer Kontrollschnittstelle (12) der Gewichtsanzeigemittel, um eine veränderte visuelle und/oder akustische Anzeige zu erzeugen, wenn eine schlechte Verteilung der Kräfte vom Erfassungsmodul (11) erfasst wird, wobei die Änderung dem Benutzer Gleichgewichtsstörungen anzeigt und den Benutzer dazu veranlasst, die Gleichgewichtsposition anzustreben.

17. Gerät nach einem der Ansprüche 1 bis 15, umfassend Mittel, um die Anzeige zu erzeugen, die den Benutzer dazu veranlasst, eine Bewegung durchzuführen.

## Claims

1. An apparatus (1) for detecting and correcting equilibrium anomalies of a human body, comprising a support plate (3) for enabling a subject to take up a position, and at least one reference system part (60) which is positionable on a substantially planar surface, the plate (3) being superposed relative to the reference system part (60), the plate (3) being supported by at least three elastic deformable members (5) each acting along a direction orthogonal to a plane defined by the reference system part, the deformable members being symmetrically distributed relative to a central axis of symmetry formed on the plate (3), the apparatus comprising:
- a processing unit (10) including a processing circuit,
**characterized in that** the apparatus further comprises:
a force sensor (4) placed at one of the ends of each of the deformable members (5), each sensor (4) delivering a signal to the processing circuit, the processing circuit being arranged for detecting an equilibrium between the signals representing the forces and corresponding to a position of equilibrium relative to a centre of symmetry of the plate (3) or detecting a disequilibrium between the signals; and
a display generating means for displaying a piece of information in response to equilibrium being detected and a modified visual and/or sound indication in response to poor distribution of the forces being detected by the processing circuit, the modified visual and/or sound indication providing an indication of equilibrium anomalies to a user, and inciting the user to seek the equilibrium position.

2. The apparatus according to claim 1, wherein the displayed information in response to detection of equilibrium is the weight of the user, the apparatus thereby defining a scale.

3. The apparatus according to claim 2, comprising:
- a module (11) for detecting disequilibrium including said processing circuit comparing data of the sensors (4) received by the processing unit (10), for detecting the poor distribution of the forces detected by each sensor (4); and
- an interface (12) for controlling the display means (2) for modifying the display when poor distribution of the forces is detected by the detection module (11).

4. The apparatus according to claim 2 or 3, including a casing (30) having a ground supporting means (6), the plate (3) being connected to the casing (30), force sensors (4) being located between said supporting means (6) and the plate (3) for deriving an electric signal representative of a force detected by the sensor (4), at least one processing unit (10) for determining from the signals derived by the sensors (4) the total weight of a subject on the scales on the plate (3), and display means (2) for indicating the determined total weight, the sensors (4) being identical and distributed in areas symmetrical to each other relative to a central axis of symmetry (S), the deformable members (5) being associated with each of the sensor areas (4) for supporting in an upward direction, the plate (3) and said sensors (4) relative to the casing (30).

5. The apparatus according to claim 4, comprising adjusting means (8, 62) for horizontally adjusting the casing (30) position.

6. The apparatus according to claim 4 or 5, wherein the ground supporting means (6) comprise four legs positioned under the casing (30), the legs being integral with a fixed base forming the reference system element (60).

7. The apparatus according to claim 3 alone or when combined with one of claims 4 to 6, wherein said control interface (12) is arranged for displaying the total weight of the subject on the scales in response to the detection module (11) deriving an indication of equilibrium being detected.

8. The apparatus according to claim 7, further comprising four elastic deformable members (5), each vertically aligned with one of the legs.

9. The apparatus according to claim 7 or 8, wherein the force sensors (4) are distributed under four corners of the plate (3) and the casing (30) includes at least one vertical member (35) connected to the reference system element (60), for limiting displacement of the plate (3) supported by the deformable members (5).

10. The apparatus according to claim 3, alone or when combined with one of claims 4 to 9, wherein the module (11) for detecting disequilibrium has a predetermined detection threshold so that the difference between the smallest and the largest of the measured forces which remains smaller than the threshold does not enable detection of disequilibrium.

11. The apparatus according to claim 3, alone or when combined with one of claims 4 to 10, comprising a selector for selecting between two operating modes:
- a conventional mode for displaying the total weight of the subject on the scales, regardless of the distribution of the forces detected by each of the sensors (4); and
- a dynamic mode for displaying the total weight of the subject on the scales, wherein said control interface (12) is arranged to prevent display of said total weight in response to a poor distribution of the forces detected by the detection module (11).

12. The apparatus according to one of claims 1 to 11, wherein the force sensors (4) are variable electric resistance sensors.

13. The apparatus according to claim 4, alone or when combined with one of claims 5 to 12, wherein a spirit level (8) is integral with the casing (30) to indicate horizontalness of the casing (30).

14. The apparatus according to one of claims 1 to 13, wherein the plate (3) bears elements for marking the location of two feet of the subject on the scale, the marking elements having at least one middle line relative to two of the force sensors (4).

15. The apparatus according to one of claims 1 to 13, comprising a short distance communications interface for transmitting data to a display means freely mobile relative to the rest of the apparatus.

16. A method of integrating into a scale (1) with a plate (3) and a visual and/or sound indication of the weight of a subject on the scale, a function for detecting equilibrium anomalies of the human body, **characterized in that** it comprises:
- symmetrically distributing force sensors (4) relative to a central axis of symmetry (S) formed on the plate of the scale (1);
- associating with each of the force sensors (4) a deformable member (5) so a portion of the plate (3) and the associated sensor (4) are supported in an upward direction;
- connecting to the sensors (4) a comparison module (11) for detecting the distribution of the forces; and
- integrating an interface (12) for controlling weight indication means so a modified visual and/or sound indication is generated in response to a poor distribution of forces being detected by the detection module (11), the modified visual and/or sound indication providing an indication of equilibrium anomalies to a user, and inciting the user to seek the equilibrium position.

17. The apparatus according to one of claims 1 to 15, comprising means for generating a display inciting the user to make a movement.
